# EUROPEAN PATENT SPECIFICATION

(11) **EP 3 749 429 B1**
(45) Date of publication and mention of the grant of the patent: **12.03.2025**
(21) Application number: 19714780.4
(22) Date of filing: 08.02.2019
(51) Int. Cl.: B01D 15/18, G01N 30/42, A61K 38/03, A61K 38/08, C07K 1/14, C07K 1/16, C07K 7/00, C07K 7/06, G01N 30/38, C12R 1/125

(54) **METHOD OF PURIFYING AND/OR SEPARATING LIPOPEPTIDE SURFACTANTS OF NATURAL ORIGIN USING COUNTERCURRENT CHROMATOGRAPHY**
VERFAHREN ZUR REINIGUNG UND/ODER TRENNUNG VON LIPOPEPTID-TENSIDEN NATÜRLICHEN URSPRUNGS MITTELS GEGENSTROM-CHROMATOGRAPHIE
PROCÉDÉ DE PURIFICATION ET/OU DE SÉPARATION DE TENSIOACTIFS LIPOPEPTIDIQUES D'ORIGINE NATURELLE AU MOYEN DE LA CHROMATOGRAPHIE À CONTRE-COURANT

(30) Priority: 09.02.2018 PL 42455518
(43) Date of publication of application: 16.12.2020
(73) Proprietor: INVENTIONBIO SP. Z O.O., 85-825 Bydgoszcz (PL)
(72) Inventor: HODUREK, Pawe, 58-309 Wa brzych (PL); JAJOR, Pawe, 57-220 Zi bice (PL); UKASZEWICZ, Marcin, 51-351 Wroc aw (PL)
(74) Representative: Kondrat, Mariusz
(86) International application number: PCT/IB2019/051017
(87) International publication number: WO 2019/155411

(56) References cited:
- US-A1- 2014 371 135
- CHEEL JOSÉ ET AL: "Separation of cyclic lipopeptide puwainaphycins from cyanobacteria by countercurrent chromatography combined with polymeric resins and HPLC", ANALYTICAL AND BIOANALYTICAL CHEMISTRY, SPRINGER, DE, vol. 409, no. 4, 30 November 2016 (2016-11-30), pages 917 - 930, XP036142785, ISSN: 1618-2642, [retrieved on 20161130], DOI: 10.1007/S00216-016-0066-Z
- JANE HUBERT ET AL: "New perspectives for microbial glycolipid fractionation and purification processes", COMPTES RENDUS - CHIMIE, ELSEVIER, PARIS, FR, vol. 15, no. 1, 8 November 2011 (2011-11-08), pages 18 - 28, XP028435858, ISSN: 1631-0748, [retrieved on 20111117], DOI: 10.1016/J.CRCI.2011.11.002
- SHAOJUN ZHANG ET AL: "Separation and purification of six biosurfactant rhamnolipids by high-speed countercurrent chromatography utilizing novel solvent selection method", SEPARATION SCIENCE AND TECHNOLOGY, vol. 51, no. 4, 3 March 2016 (2016-03-03), pages 673 - 680, XP055594319, ISSN: 0149-6395, DOI: 10.1080/01496395.2015.1117101

## Description

The subject-matter of the invention is a method of purifying and/or separating lipopeptide surfactants of the natural origin using the Countercurrent Chromatography (the CCC) techniques, for example by means of the centrifugal partition chromatography, which is a hydrostatic variation of the CCC, in the phasal arrangements involving the use of the aqueous buffer solutions, liquid aliphatic alcohols and liquid aliphatic hydrocarbons, in which homologues represent partition ratios from 0.1 to 10. The method of purifying the lipopeptide surfactants of the natural origin using the Countercurrent Chromatography techniques, e.g. the Centrifugal Partition Chromatography, may also be conducted with or without partition into homologues.

Lipopeptide surfactants of the natural origin are organic substances produced by many different groups of organisms and that are also present in the (envelopes/coatings) of certain viruses. However, effectiveness of lipopeptide surfactants differs between various groups of organisms. Currently, certain bacteria are recognized as the most effective in producing the said surfactants under specific conditions. For example, *Bacillus* sp. (e.g. *Bacillus subtilis)* produces various forms of surfactin (both its analogues and homologues), or *Pseudomonas* sp. (e.g. *Pseudomonas fluorescens* BD5) produces various forms of pseudofactin.

Lipopeptide surfactants of the natural origin are very efficient surfactants and emulsifiers. They are also much more effective than for example soap. Unlike synthetic detergents, they are fully biodegradable, and, therefore, not as burdensome for the natural environment as synthetic detergents. So far, they have not been identified as highly toxic for human body, yet, they show a bacteriostatic, and sometimes a bacteri-, and fungicidal effect. Due to these characteristics, natural lipopeptide surfactants may be used as emulsifiers in creams, while reducing the required quantity of preservative agents or fully eliminating the use of thereof, or, they can be applied in e.g. modern dishwashing liquids and other cleaners.

For many years, biosurfactants have been isolated (and purified) using different methods that have not always been efficient. For example, surfactin is quite often isolated from *Bacillus subtilis* using acidic precipitation. This is the most common method of obtaining surfactin, which, however, entails losses amounting to 50% and, sometimes, the obtained surfactin is contaminated with e.g. free fatty acids.

The Countercurrent Chromatography is a preparative chromatographic technique that is based on separation between two liquids that are immiscible with each other. The Centrifugal Partition Chromatography is one of the technical solutions of a so-called hydrostatic type, where retention of the stationary phase is maintained by means of the hydrostatic pressure that is generated by a single-axis centrifuge equipped with rotation seals. Like other methods of Countercurrent Chromatography, the Centrifugal Partition Chromatography is considered no-, or low-loss technique. Absence of adsorption effects on the deposit guarantees a high ratio of recovery from the apparatus (above 90%). At the same time, the CPC is a chromatographic method that makes it possible to get rid of unwanted contamination in an effective manner.

The separation of a biosurfactant mixture into components (e.g. structural homologues) is yet another issue. So far, this has been achieved using the preparative High Performance Liquid Chromatography (prepHPLC), which, however, is not efficient. It is a costly technique that requires a large amount of solvents of high-purity grade. The Countercurrent Chromatography techniques consume less solvents per one gram of pure substance and, in addition, they allow using solvents with a lower purity grade. Furthermore, as far as the centrifugal partition chromatography is concerned, the chromatographic resolution is not lost in the event of transition into a larger scale; the phenomenon is strongly highlighted, as far as the prepHPLC is concerned.

The Centrifugal Partition Chromatography has been getting more and more popular among the modern purification methods over the years. In 1993, André Durand et al. [WO 1994021622], filed a patent application for taxoid purification method using CPC. In 2010, Matthieu Giraud et al. [WO 2011157803], filed a patent application for a method of purifying atmospheric compounds using specific ionic exchangers. In 2013, a patent application was filed for a methodology for purifying oligonucleotides from a mixture using countercurrent chromatography techniques, including CPC [WO 2013030263]. In 2016, Cheel José et al. [Cheel, J.; Urajová, P.; Hájek, J.; Hrouzek, P.; Kuzma, M.; Bouju, E.; Faure, K.; Kopecký, J. Separation of cyclic lipopeptide puwainaphycins from cyanobacteria by countercurrent chromatography combined with polymeric resins and HPLC. Anal. Bioanal. Chem. 2017, 409, 917- 930] reported a method of purifying puwainaphycins (a group of β-amino acid cyclic lipopeptides) from cyanobaterial biomass using countercurrent chromatography techniques, including high-performance countercurrent chromatography HPCCC into two fractions using the system n-hexane : ethyl acetate : ethanol : water in the proportion of 1:5:1:5, by volume. Fraction I was subjected by HPCCC, using the system ethyl acetate : ethanol : water (5:1:5, by volume), and fraction II was subjected by HPCCC, using the system n-hexane : EtOAc : ethyl acetate : water (1:5:1:5, by volume). In both cases, the lower phase were employed as mobile phases. As a result, three new puwainaphycin variants with hydroxy- or chloro- substitution on their fatty acid side chains are reported for the first time. Recently, more and more techniques of purification of subsequent chemical compounds have been patented, for instance, in 2017, Rotachrom company filed a patent application for other methods employing CPC for purification of, e.g. baccatine III, or cyclosporin A [WO 2017072542 A1, WO 2017098291]. The method of purification of lipopeptide surfactants of the natural origin using the Centrifugal Partition Chromatography falls within the trend followed by an increasing number of patent applications employing the CPC techniques.

The invention relates to a method of purifying and/or separating surfactin using the Countercurrent Chromatography (CCC), characterised in that: the purification occurs within the system of two partially mixed solvent phases prepared using solvents selected from a group comprising aqueous buffer solutions, liquid aliphatic alcohols and liquid aliphatic hydrocarbons, whereby the buffer solutions have their pH stabilized within the range of 2 to 10 and the concentrations of the buffering ions fall within the range from 2 to 800 mM; aliphatic hydrocarbons contain from 5 to 12 carbon atoms; wherein said surfactants represent partition ratios within the range from 0.1 to 10 in said solvent systems, wherein both solvent phases comprise a mixture of aqueous buffer solution : methanol : n-Butanol : n-Hexane is applied in the proportion of 3:2:3:2, by volume, and wherein said aqueous buffer solution contains a disodium phosphate, sodium sulphate and disodium edetate; methanol; n-Butanol; and n-Hexane.

Preferably, purification and/or separation of the lipopeptide surfactants of the natural origin is carried out using the Centrifugal Partition Chromatography (CPC), which is one of the versions of the Countercurrent Chromatography (CCC) of the hydrostatic type.

Preferably, additional stabilising components, preferably auxiliary salts, are employed in aqueous buffer solutions.

Like in any Countercurrent Chromatography technique, in the Centrifugal Partition Chromatography, a (solvent) system is applied that constitutes a unique composition of two liquid phases that are in the state of equilibrium with respect to each other and between which the process of countercurrent chromatography can take place.

Purification of the lipopeptide surfactants with or without partition into homologues may be carried out in a few operation modes: an ascending mode (abbreviation: ASC), which is the operational mode that allows employing a heavier fluid as the stationary phase and a lighter fluid as the mobile phase. Then, in the CPC in the ASC mode, a flow through the partition cells occurs in the direction towards the axis of rotation of the impeller, that is opposite to the centrifugal force.

A descending mode (abbreviation: DSC) is the operation mode that allows employing the lighter phase as the stationary phase and the heavier phase as the mobile phase. Then, in a CPC in the DSC mode, a flow through the partition cells takes place in the direction from the axis of rotation of the impeller, that is concurring with the centrifugal force.

The dual-mode (abbreviation: DM) is an operating mode consisting in the mobile and stationary phases changing places with each other with a view to, e.g. increasing the chromatographic resolution (i.e.: the ASC coming first, and the DSC later on (or vice versa)).

The multiple dual-mode (abbreviation: MDM) is an operating mode that consists in the mobile and stationary phases changing places with each other a multiple times.

The solvent system for purifying the lipopeptide surfactants of the natural origin is prepared using the following solvents: buffer solutions that have pH stabilised within the range from 2 to 10 and the buffering ion concentrations falling within the range from 2 to 500 mM, whereby the aqueous buffer solutions may contain additional stabilising components, preferably auxiliary salts (that introduce an appropriate concentration of the accompanying ions and moderate the ion strength, such as e.g. NaCl); simple aliphatic branched or cyclic hydrocarbons, with the number of carbons from 1 to 10.

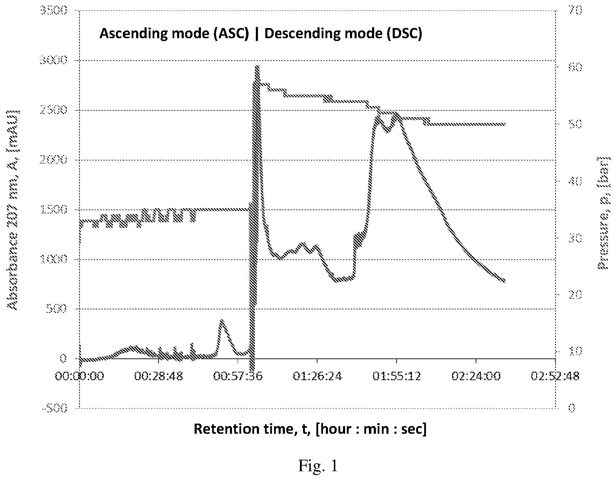

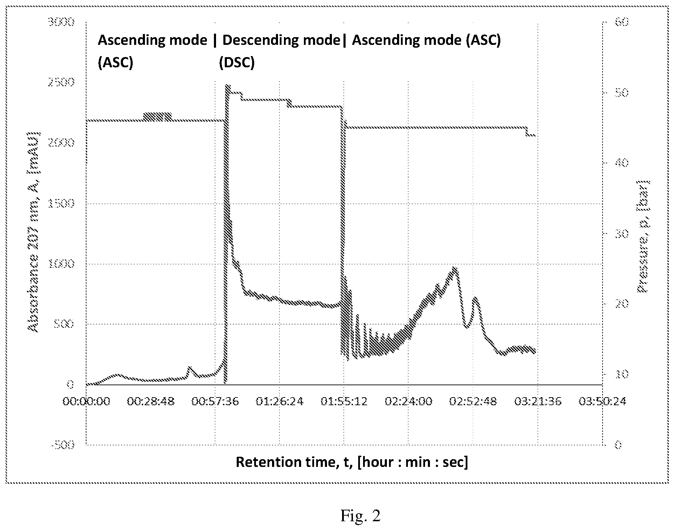

The invention has been presented in detail in the following examples and in the Figure, where fig. 1 presents an exemplary chromatogram for purification of a surfactin obtained from the *Bacillus subtilis,* without separation into homologues, and fig. 2 presents purification of a surfactin obtained from the *Bacillus subtilis,* involving the separation into homologues.

### Example 1. Purification of a surfactin obtained from the Bacillus subtilis, without separation into homologues:

The system components that have been used:
1. Aqueous buffer solution containing: 20 mM of disodium phosphate, 50 mM of sodium chloride and 5 mM of disodium edetate (disodium EDTA);
2. Methanol,
3. n-Butanol; and
4. n-Hexane.

The solvent system was prepared in the proportion of 3:2:3:2, respectively, with the sequence of the components as presented above.

### Sample preparation:

Approx. 1.2 g of a purified preparation was suspended in 40 ml of the above-described solvent system (with the same quantity of the upper and lower phase) and the remains were centrifuged for 10 min with the acceleration of at least 1500 g and not greater than 2500 g; the supernatant was transferred into a clean container and centrifuged again for a short time (approx. one minute, 1500 g) so that the phases become separated.

### The module employed for the CPC:

Centrifuge with an impeller with the capacity of 1 litre and the separated cells in the quantity of at least 1800, e.g.: Armen SCPC 1 L.

### Preparation of the chromatographic column was presented in Table 1.

**Table 1.**

| Duration of the action [min : s] | The elution mode (position of the valve) | Heavier phase (percentage share) % | Lighter phase (percentage share) % | Flow [ml/min] | Impeller rotations [RPM] |
|---|---|---|---|---|---|
| 0:00 | **ASC** | 100 | 0 | 100 | 500 |
| 13:00 | **ASC** | 100 | 0 | 100 | 500 |
| 13:03 | **ASC** | 0 | 100 | 30 | 1500 |
| 35:00 | **ASC** | 0 | 100 | 30 | 1500 |
| 38:00 | **ASC** | 0 | 100 | 8 | 1500 |
| 40:00 | **ASC** | 0 | 100 | 8 | 1500 |

Approx. 20 ml of a preparation dissolved in the system phases is injected into a column so prepared.

Elution programme, the version involving filling in the impeller and without extrusion, in accordance with Table 2 as presented below:

**Table 2**

| Duration of the action [h : min : s] | The elution mode (position of the valve) | Heavier phase (percentage share) % | Lighter phase (percentage share) % | Flow [ml/min] | Impeller rotations [RPM] |
|---|---|---|---|---|---|
| 0:00 | **ASC** | 0 | 100 | 8 | 1500 |
| 05:00 | **ASC** | 0 | 100 | 8 | 1500 |
| 35:00 | **ASC** | 0 | 100 | 15 | 1500 |
| 01:02:00 | **ASC** | 0 | 100 | 15 | 1500 |
| 01:02:03 | **DSC** | 100 | 0 | 12 | 1500 |
| 02:40:00 | **DSC** | 100 | 0 | 12 | 1500 |

Fractions should be collected into a collector within the range of time from 1:35:00 to 2:40:00, whereby the absorbance at a wavelength from the range of 205 to 220 nm, e.g. at 207 nm, is monitored.

The purity of the surfactin so obtained amounts to ≥90%, whereby obtained surfactin is a mixture of homologues.

### Example 2. Purification of a surfactin that is obtained from the Bacillus subtilis bacteria involving separation into homologues:

Homologues are a group of similar chemical compounds that form a so-called "homologous series," in which subsequent compounds differ from the previous ones by a methylene group (link) (CH₂).

The system components that have been used:
1. Aqueous buffer solution containing: 20 mM of disodium phosphate, 50 mM of sodium chloride and 5 mM of disodium edetate (disodium EDTA);
2. Methanol,
3. n-Butanol; and
4. n-Hexane.

The solvent system should be prepared in the following proportion: 3:2:3:2 of the above components, respectively.

### Sample preparation:

Approx. 1.2 g of the preparation being purified should be suspended in 40 ml of the system (with the same quantity of the upper and lower phase). After the suspending, the remains should be centrifuged for 10 min, with the acceleration ranging from at least 1500 g and not exceeding 2500 g. The supernatant should be transferred into a clean container, and centrifuged again for a short time so that the phases become separated.

### The module employed for the CPC:

Centrifuge with an impeller with the capacity of 1 litre and the separation cells in the quantity of at least 1800, e.g.: Armen SCPC 1 L.

Preparation of the chromatographic column in accordance with Table 3, as follows:

**Table 3**

| Duration of the action [min : s] | The elution mode (position of the valve) | Heavier phase (percentage share) % | Lighter phase (percentage share) % | Flow [ml/min] | Impeller rotations [RPM] |
|---|---|---|---|---|---|
| | | | | | |
| 0:00 | **ASC** | 100 | 0 | 100 | 500 |
| 13:00 | **ASC** | 100 | 0 | 100 | 500 |
| 13:03 | **ASC** | 0 | 100 | 30 | 1500 |
| 35:00 | **ASC** | 0 | 100 | 30 | 1500 |
| 38:00 | **ASC** | 0 | 100 | 8 | 1500 |
| 40:00 | **ASC** | 0 | 100 | 8 | 1500 |

Approx. 5 ml of the preparation is injected into a column so prepared; whereby the upper phase upon the injection is preferred.

Elution programme, the version involving filling in the impeller and without extrusion, in accordance with Table 4 as presented below:

**Table 4.**

| Duration of the action [h : min : s] | The elution mode (position of the valve) | Heavier phase (percentage share) % | Lighter phase (percentage share) % | Flow [ml/min] | Impeller rotations [RPM] |
|---|---|---|---|---|---|
| 0:00 | **ASC** | 0 | 100 | 8 | 1500 |
| 05:00 | **ASC** | 0 | 100 | 8 | 1500 |
| 35:00 | **ASC** | 0 | 100 | 15 | 1500 |
| 01:02:00 | **ASC** | 0 | 100 | 15 | 1500 |
| 01:02:03 | **DSC** | 100 | 0 | 12 | 1500 |
| 01:54:00 | **DSC** | 100 | 0 | 12 | 1500 |
| 01:54:03 | **ASC** | 0 | 100 | 12.0 | 1500 |
| 03:20:00 | **ASC** | 0 | 100 | 21.5 | 1500 |

Fractions should be collected into a collector within the range of time from 02:20:00 to 03:10:00, whereby the absorbance at a wavelength from the range of 205 to 220 nm, e.g. at 207 nm, is monitored. The amount of the collected fractions should fall within the range of 15-20 ml.

However, the surfactin homologues separated with this method are still contaminated with each other. Only certain selected fractions achieve purity at the level of 80% of specific homologue. The foregoing is mainly true of the C13, C14 and C15 homologues. Therefore, the selected fractions should be analytically examined, namely those which (following a review of the CPC chromatogram) seem interesting. Only those where the purity (from other homologues) is satisfactory (e.g. ≥75%) should be selected for further research.

It should also be borne in mind that the purity of the surfactin so obtained as a whole (without the separation into homologues being taken into account) amounts to ≥ 90%.

## Claims

1. A method of purifying and/or separating surfactin using Countercurrent Chromatography (CCC), **characterised in** t**hat**: the purification occurs within the system of two partially mixed solvent phases which comprises a mixture of an aqueous buffer solution : methanol : n-Butanol : n-Hexane in the proportion of 3:2:3:2, by volume,
wherein said aqueous buffer solution contains disodium phosphate, sodium chloride and disodium edetate;
whereby the buffer solution have its pH stabilized within the range of 2 to 10 and the concentrations of the buffering ions fall within the range from 2 to 800 mM; wherein said surfactin represents partition ratios within the range from 0.1 to 10 in said solvent systems.

2. The method of claim 1, **characterised in that** it is carried out using the Centrifugal Partition Chromatography (CPC).

3. The method of claim 1, **characterised in that**, additional stabilising components, preferably auxiliary salts are employed in aqueous buffer solutions.

## Patentansprüche

1. Verfahren zur Reinigung und/oder Abtrennung von Surfaetin unter Verwendung von Gegenstromchromatographie (CCC), **dadurch gekennzeichnet, dass**: die Reinigung innerhalb des Systems von zwei teilweise gemischten Lösungsmittelphasen erfolgt, das eine Mischung aus einer wässrigen Pufferlösung : Methanol : n-Butanol : n-Hexan im Volumenverhältnis 3:2:3:2 umfasst,
wobei die wässrige Pufferlösung Dinatriumphosphat, Natriumchlorid und Dinatriumedetat enthält;
wobei der pH-Wert der Pufferlösung im Bereich von 2 bis 10 stabilisiert ist und die Konzentrationen der Pufferionen im Bereich von 2 bis 800 mM liegen; wobei das Tensid Verteilungsverhältnisse im Bereich von 0,1 bis 10 in den Lösungsmittelsystemen aufweist.

2. Verfahren nach Anspruch 1, **dadurch gekennzeichnet, dass** es unter Verwendung der Zentrifugalpartitionschromatographie (CPC) durchgeführt wird.

3. Verfahren nach Anspruch I, **dadurch gekennzeichnet, dass** in wässrigen Pufferlösungen zusätzliche stabilisierende Komponenten, vorzugsweise Hilfssalze, eingesetzt werden.

## Revendications

1. Méthode de purification et/ou de séparation de la surfaétine par chromatographie à contre-courant (CCC), **caractérisée en ce que** : la purification a lieu dans le système de deux phases de solvant partiellement mélangées qui comprend un mélange d'une solution tampon aqueuse : méthanol : n-Butanol : n-Hexane dans la proportion de 3:2:3:2, en volume,
dans laquelle ladite solution tampon aqueuse contient du phosphate disodique, du chlorure de sodium et de l'édétate disodique ;
La solution tampon a un pH stabilisé entre 2 et 10 et concentrations des ions tampons sont comprises entre 2 et 800 mM ladite surfactine représente des rapports de partage compris entre 0,1 et 10 dans lesdits systèmes de solvants.

2. La méthode de la revendication 1, **caractérisée par le fait qu'**elle est réalisée à l'aide de la chromatographie de partage centrifuge (CPC).

3. La méthode de la revendication I, **caractérisée par le fait que** des composants stabilisants supplémentaires, de préférence des sels auxiliaires, sont utilisés dans les solutions tampons aqueuses.
